# EUROPEAN PATENT SPECIFICATION

(11) **EP 1 954 340 B1**
(45) Date of publication and mention of the grant of the patent: **17.07.2019**
(21) Application number: 06770478.3
(22) Date of filing: 16.05.2006
(51) Int. Cl.: A61M 25/10, A61M 25/00, B29C 49/00, B29K 105/00, B29L 31/00, B29C 49/04, B29C 49/48, B29C 67/00

(54) **BALLOON FOLDING DESIGN**
BALLONFALTDESIGN
MECANISME DE PLIAGE DE BALLON

(30) Priority: 14.11.2005 US 273330
(43) Date of publication of application: 13.08.2008
(73) Proprietor: Boston Scientific Limited, Hamilton HM11 (BM)
(72) Inventor: ROMAN, Ricardo, D., San Diego, CA 92139 (US)
(74) Representative: Vossius & Partner Patentanwälte Rechtsanwälte mbB
(86) International application number: PCT/US2006/019054
(87) International publication number: WO 2007/055732

(56) References cited:
- WO-A-97/25093
- WO-A-99/44649
- US-A- 6 013 055
- US-A- 6 024 722
- US-A1- 2004 133 223
- US-B1- 6 544 224

## Description

### BACKGROUND OF THE INVENTION

Atherosclerotic cardiovascular disease is common, and is caused by a narrowing of the artery due to atherosclerotic plaques. Medical balloons are used in the body in a variety of applications including as dilatation devices for compressing plaque and for expanding prosthetic devices such as stents at a desired location in a bodily vessel.

Percutaneous transluminal angioplasty (including percutaneous transluminal coronary angioplasty (PTCA)), or balloon angioplasty, is a non-invasive, non-surgical means of treating peripheral and coronary arteries. This technique consists of inserting an uninflated balloon catheter into the affected artery. Dilation of the diseased segment of artery is accomplished by inflating the balloon which pushes the atherosclerotic lesion outward, thereby enlarging the arterial diameter.

Another type of medical balloon are those having cutting edges, also referred to as atherotomes or blades, for recanalizing and dilating a diseased vessel, and facilitating balloon angioplasty procedures. Balloons may also be equipped with injectors or other surface features as well.

For such applications, the balloon traverses a tortuous anatomy as it is being delivered to the location in a bodily vessel; it is desirable for the balloon to assume as low a profile, i.e. the outer diameter of the distal end portion of the balloon, as possible. Considerable effort has been put forth in the development of dilatation balloons with a low profile by, for example, minimizing the dimensions of the core or the inner tube which extends through the balloon to its distal end, and by reducing the wall thickness of the balloon itself.

The folding of medical balloons in order to reduce the cross sectional area or profile of the balloon in a deflated state of the balloon is a common industry practice. Balloons may also be folded in order to enhance re-fold characteristics of the balloon during deflation.

Folding of the balloon often involves formation of a number of wings in the balloon. In the deflated state, the balloon collapses upon itself forming flaps or wings that must be folded or wrapped about the balloon catheter to allow it to be withdrawn from the patient's vasculature after use.

Also prior to use, the balloon is typically folded or wrapped about the balloon catheter to fit within and pass through the guide catheter lumen. When inflation fluid is applied to the deflated balloon, the balloon wings or flaps unwrap or unfold and the balloon inflates to a fully expanded condition.

Various techniques or balloon constructions have been employed to facilitate the folding of the balloon about the balloon catheter in a uniform manner upon evacuation and deflation of the balloon after use.

There remains a need, however, for innovative and improved methods for folding balloons and for improved balloon refold. The present invention is directed to this need.

US 2004/0133223A1 discloses medical devices such as medical balloons, catheters having balloons and stents, having one or more cutting elements on their outer surface.

It discloses a medical balloon having a body section, a first tapered section and a second tapered section, waist sections, and a longitudinal axis, said balloon having a deflated state and an inflated state, the body section of said balloon having a plurality of concave side regions extending between the first tapered section and the second tapered section, each concave side region curving toward the longitudinal axis of the balloon, the balloon comprising a plurality of atherotomes (cutting elements), each atherotome located centrally in each concave side region, the body section having a circumference which when viewed in radial cross-section, has at least one layer extending around the entire circumference and each said layer is formed from a substantially homogeneous polymer material, and in the expanded state the body section of said balloon is circular in cross-section.

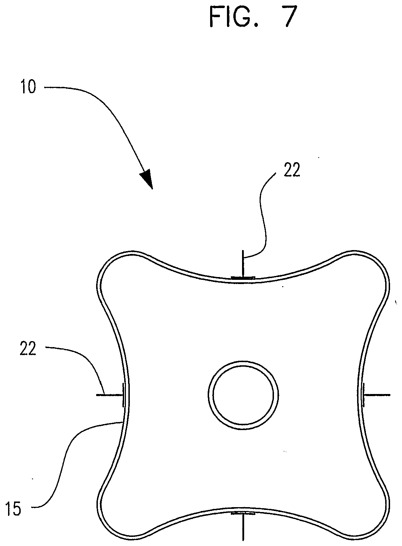

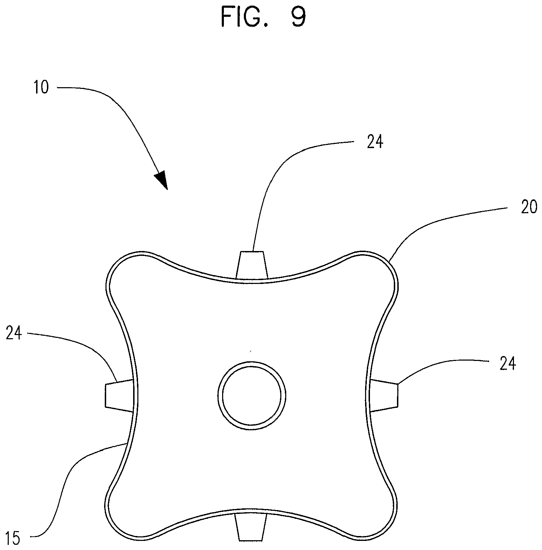

Figs. 7 to 9 disclose a catheter having four cutting elements. The balloon can be folded to protect the cutting elements. The balloon is coextruded having a circular cross- section as shown in Fig. 9.

The problem of the invention is to provide a balloon carrying atherotomes, wherein the balloon has improved folding and refolding characteristics.

The problem is solved by the balloon according to claim 1.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### SUMMARY OF THE INVENTION

Embodiments of the present invention related to medical balloons which may be employed in combination with catheter assemblies for a variety of purposes such as for angioplasty, and for implantable medical device delivery such as stent delivery. Stents may be employed in the coronary arteries, peripheral vasculature, cerebrum, urethra, ureter, gastrointestinal tract, esophagus, urinary tract, bile ducts, alimentary tract, tracheobronchial tree, cerebral aqueducts, genitourinary system, prostatic urethra, fallopian tubes, as well as other regions of the body.

In at least one embodiment, a medical balloon having a body section, first and second tapered sections, and waist sections is formed such that in its static state, at least the body section has a plurality of concave side regions which extend between the first and second tapered sections. The concave side regions curve toward the
longitudinal axis of the balloon. In one embodiment, the first and second tapered sections also have corresponding concave side regions.

In at least one embodiment, the concave side regions curve toward the longitudinal axis such that the center portion of the concave side regions is closest to the longitudinal axis.

In some embodiments the body section of the balloon further has a circumference which when viewed in radial cross-section, has at least one layer extending around the entire circumference which is formed from a homogeneous polymer material. In some embodiments the balloon comprises multiple layers.

In at least one embodiment, in the expanded state, the body section of the balloon, as well as the tapered sections, may take on a substantially cylindrical configuration.

The balloon according to the invention is formed in a mold form having an interior cavity respectively shaped to define corresponding portions of said balloon. The balloon exiting the mold form is in its static state.

The invention is defined by the appended claims. Subject matter referred to as embodiments and/or inventions which are not claimed are not part of the invention.

The balloon according to the invention is formed in a mold form which is configured such that the mold cavity has a body section, first and second tapered sections, and waist sections, at least the body section has a plurality of concave side regions which extend between the first and second tapered sections. The concave side regions curve toward the longitudinal axis of the mold form. In one embodiment, the first and second tapered sections also have corresponding concave side regions.

The balloon can be deflated from a static state and folded. The balloon is particularly adapted for inflation from its folded configuration to an expanded configuration, and then deflated, and back to the folded configuration. The balloon according to the invention is designed for improved folding and refolding characteristics.

These and other aspects, embodiments and advantages of the present invention will be apparent to those of ordinary skill in the art upon review of the Detailed Description and Claims to follow.

### BRIEF DESCRIPTION OF THE DRAWINGS

FIG. 1 is a perspective view of one example of a balloon according to the invention shown in a static state.
FIG. 2 is a radial cross-section taken at section 2 in FIG. 1 shown in a static state.
FIG. 3 is a radial cross-section of a balloon similar to that shown in FIGS. 1 and 2 in a fully expanded state.
FIG. 4 is a radial cross-section of a balloon similar to that shown in FIGS. 1 through 3 in a partially deflated state.
FIG. 5 is a radial cross-section of a balloon similar to that shown in FIGS. 1 through 4 partially folded or wrapped.
FIG. 6 is a radial cross-section of a balloon similar to that shown in FIGS. 1 through 5 in a folded or wrapped configuration.
FIG. 7 is a radial cross-section of another embodiment of a balloon according to the present invention further having atherotomes.
FIG. 8 is a radial cross-section of a balloon similar to that shown in FIG. 7 in a partially deflated state.
FIG. 9 is a radial cross-section of another embodiment of a balloon according to the present invention further having drug delivery cones or injectors.
FIG. 10 is a radial cross-section of a balloon similar to that in FIG. 9 in a partially deflated state.
FIG. 11 is a radial cross-section of a balloon similar to that in FIGS. 9 and 10 in a folded or pleated state.
FIG. 12 is a perspective view of an alternative example of a balloon according to the invention having three concave sides in a static state.
FIG. 13 is a radial cross-section taken at section 13 in FIG. 12.
FIG. 14 is a radial cross-section of a balloon similar to that in FIGS. 12 and 13 in a partially deflated state.
FIG. 15 is a radial cross-section of a balloon similar to that shown in FIGS. 12 to 14, in a partially folded or wrapped state.
FIG. 16 is a radial cross-section of a balloon similar to that shown in FIGS. 12 to 15 in a fully folded or wrapped state.
FIG. 17 is a radial cross-section of a balloon similar to that shown in FIGS. 12 to 16 in a fully expanded state.
FIG. 18 is a perspective view of one-half of a balloon mold.
FIG. 19 is a radial cross-section of one-half of a balloon mold taken at section 19 in FIG. 18.
FIG. 20 is a longitudinal cross-sectional view of an expandable medical balloon in combination with a catheter assembly.

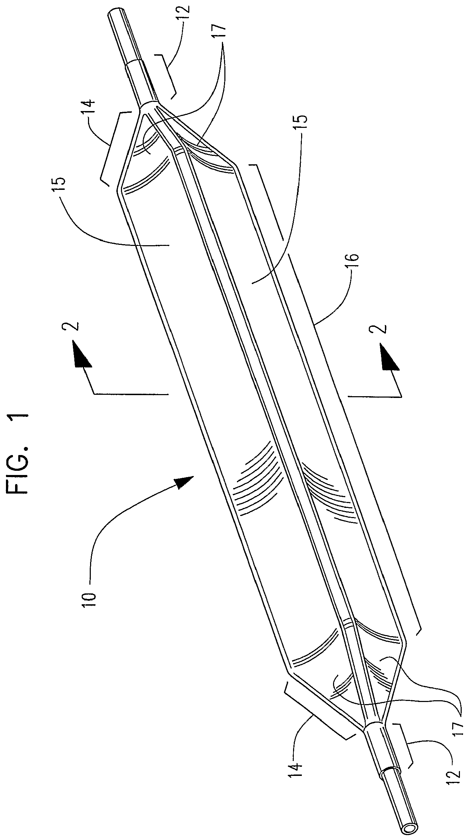

Figs. 1 - 6, 9 - 20 do not form part of the invention.

### DETAILED DESCRIPTION OF THE PREFERRED EMBODIMENTS

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

Depicted in the figures are various aspects of the invention. Elements depicted in one figure may be combined with, or substituted for, elements depicted in another figure as desired.

A medical balloon according to the present invention includes an inflation chamber formed by a balloon wall.

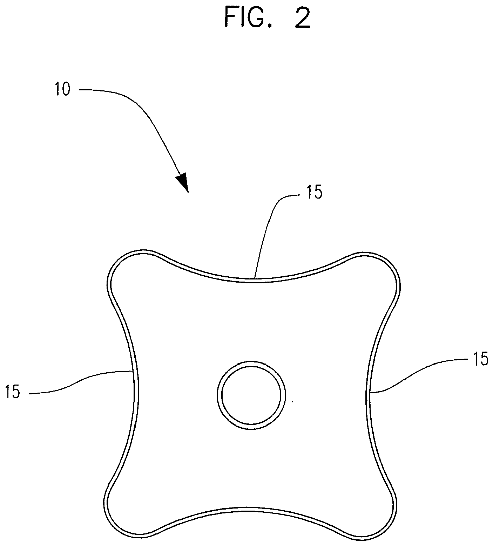

Turning now to the figures, FIG. 1 represents medical balloon 10 according to the invention. Balloon 10 is adapted to be folded into a predetermined configuration. Balloon 10 is shown in FIG. 1 in a static state. As used herein, the static state of the balloon shall refer to the balloon as formed such as immediately after removal from a mold after a blow molding process, for example, and prior to any deflation or inflation. Balloon 10 is shown having a plurality of concave side regions 15. In this particular embodiment, balloon 10 is shown having 4 concave side regions 15 in a static state. Balloon 10 is also shown having waist 12, cone regions 14 and body. In this embodiment, cones 14, in addition to body 16, are shown having concave regions 17 located in cone regions 14. One or more of these concave side regions are for facilitating balloon folding and refolding. FIG. 2 is a radial cross-section of balloon 10 taken at section 2 in FIG. 1.

A balloon may be deflated from its static state, and folded or wrapped about the shaft of a catheter assembly (not shown).

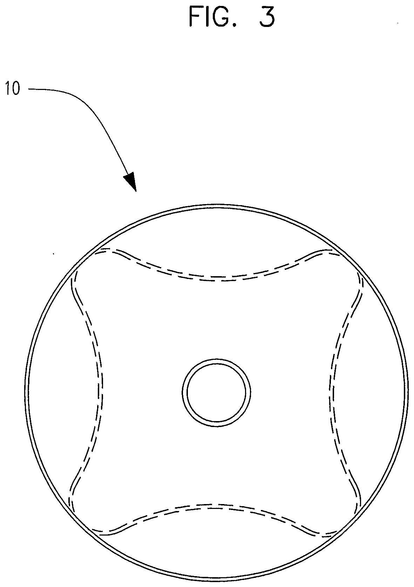

Balloon 10 is adapted for inflation from a folded configuration to an inflated, expanded configuration and back to a folded configuration. FIG. 3 is a radial cross-sectional view representing balloon 10 in an inflated state. Phantom lines indicate the balloon 10 in its static state prior to inflation.

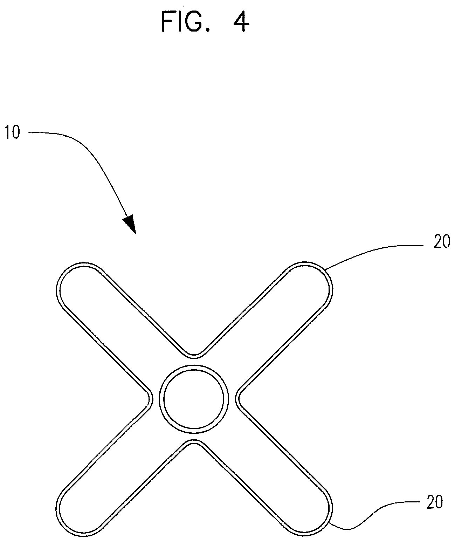

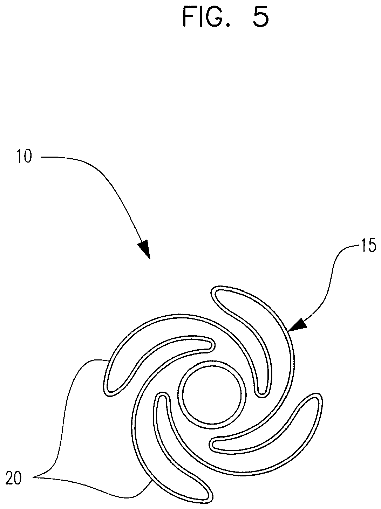

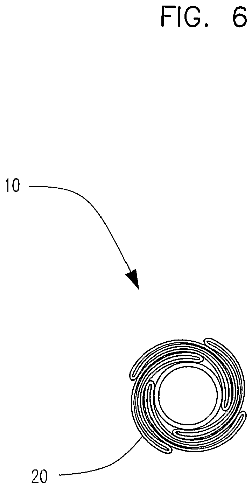

FIG. 4 is a radial cross-sectional view representing balloon 10 in a partially deflated state, prior to folding. As can be seen from FIG. 4, balloon 10 in this partially deflated state, has four wings 20. FIG. 5 is a radial cross-sectional view representing balloon 10 in a partially folded or wrapped state, and FIG. 6 is a radial cross-sectional view showing balloon 10 in a fully folded or wrapped state using methods known in the art.

In a typical folding procedure, the balloon may be placed in a folding apparatus, negative pressure applied, and the balloon manipulated with impinging members or blades from the folding apparatus into its folded or wrapped state.

After folding of the balloon, a balloon protector (not shown) may be added and the resultant assembly may then be sterilized.

Balloon 10 constructed and arranged similarly as those shown in FIGS. 1 to 6 above, is ideally suited for the addition of other features such as blades or atherotomes, for example. FIG. 7 is a radial cross-sectional depiction of an embodiment of a balloon 10 shown in a static state wherein atherotomes 22 have been added centrally to each of the concave side regions 15. Such balloon designs can be employed for recanalization and dilation of diseased vessels, and for facilitating balloon angioplasty procedures. The balloons according to the invention may also be equipped with injectors or other surface features as well.

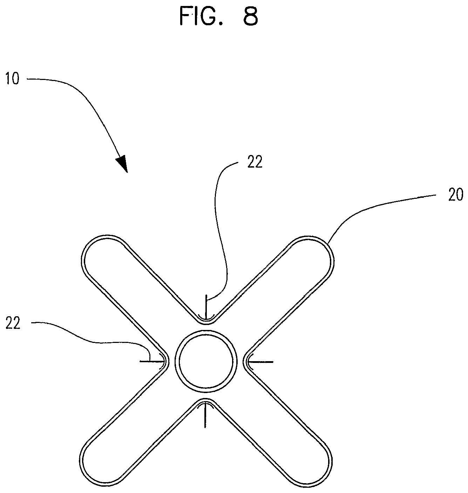

FIG. 8 is a radial cross-section showing a balloon 10 similar to that shown in FIG. 7 in a partially deflated state, prior to folding.

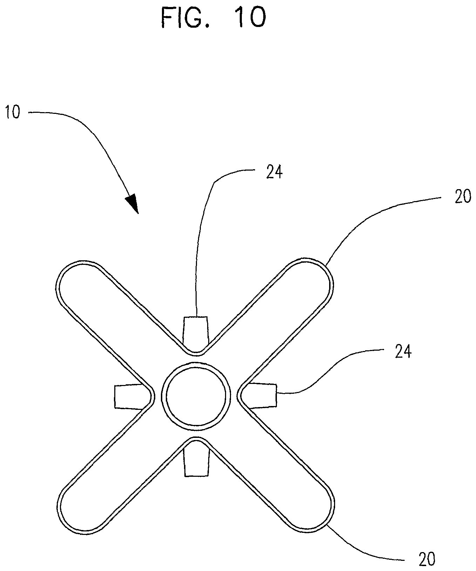

FIG. 9 is a radial cross-section of an alternative example of a balloon 10 shown in a static state. Balloon 10 is shown having drug delivery cones or injectors 24. Drug delivery cones or injectors 24 are shown located centrally of concave side regions 15 of balloon 10. FIG. 10 is a radial cross-section showing a balloon 10 similar to that shown in FIG. 9 in a partially deflated state, prior to folding.

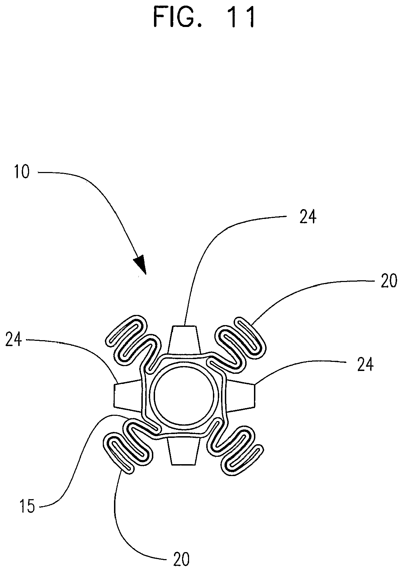

FIG. 11 is a radial cross-section of a balloon similar to that in FIGS. 9 and 10 in one example of a folded or pleated state.

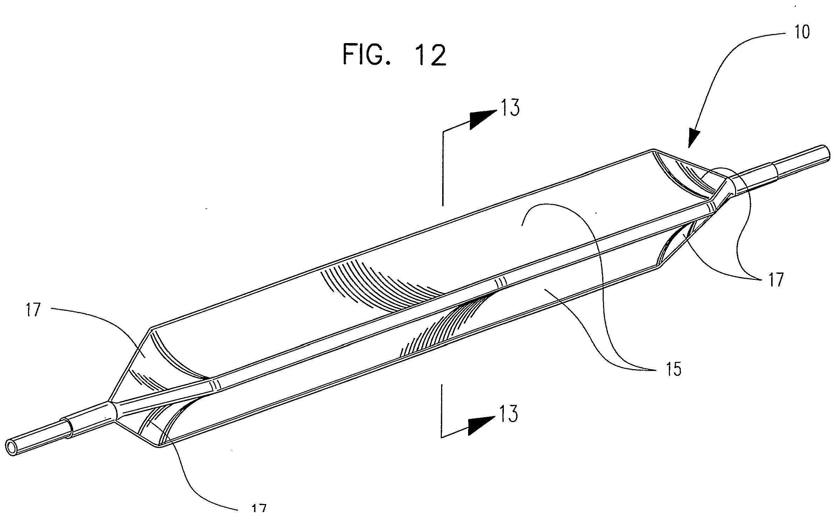

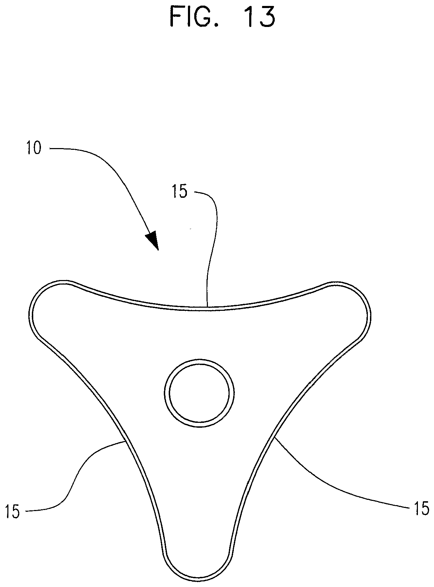

FIG. 12 is a perspective view of an alternative example of balloon 10 shown having a plurality, in this embodiment three, of concave sides 15. FIG. 13 is a radial cross-sectional view of balloon 10 taken at section 13 in FIG. 12.

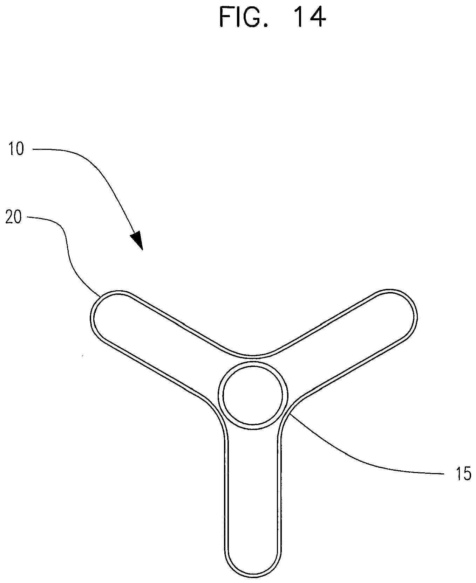

FIG. 14 is a radial cross-section of a balloon similar to that shown in FIGS. 12 and 13 in a partially deflated state prior to folding or wrapping.

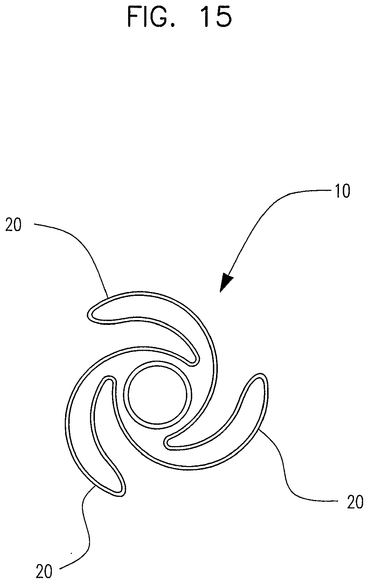

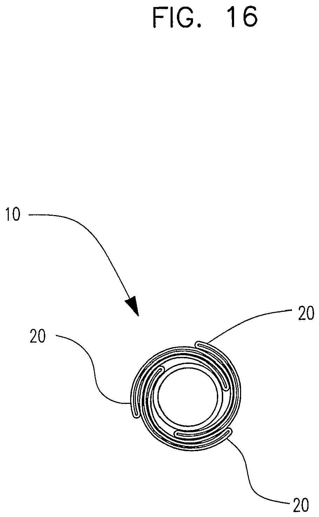

FIG. 15 is a radial cross-section of a balloon similar to that shown in FIGS. 12 to 14 in a partially deflated state and in a partially folded or wrapped state about catheter shaft while FIG. 16 is a radial cross-section of a balloon similar to that in FIGS. 12 to 15 fully folded or wrapped around shaft.

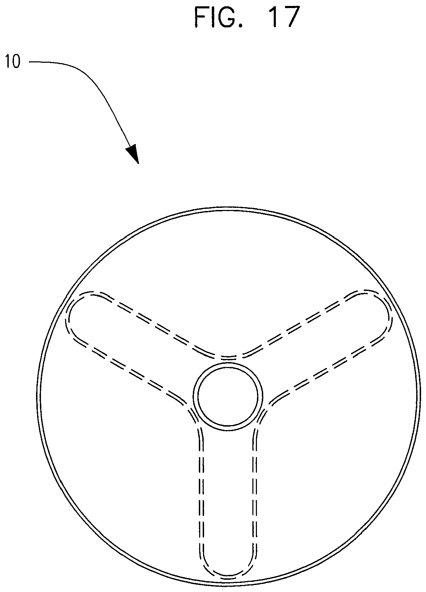

FIG. 17 is a radial cross-section of a balloon similar to those shown in FIGS. 12 to 16 in a fully expanded state.

For any of the above examples and the embodiment showen in Fig. 7 and Fig. 8, any suitable balloon folding apparatus may be employed herein. The above is only one exemplification of a balloon folding method and is not intended to limit the scope of the present invention. Balloon folding apparatuses and techniques are known in the art. For example, balloon folding apparatuses and methods thereof are discussed in commonly assigned co-pending U.S. Published Application Nos. 2003/0083687A1 and 2003/0163157A1.

Other balloon folding apparatuses and methods thereof are described in US 5350361, US 6126652, US 2002/0163104A1, US 6033380, to mention only a few. The present invention is not limited by the type of balloon folding apparatus or method used therein.

Once folded, the present invention typically does not require heat setting of the balloon, although this step does not have to be excluded and in some embodiments it may be desirable to employ a heat set.

Balloon protectors or sleeves may be employed to keep the balloon folded prior to inflation and to help refold the balloon during and after deflation. One example is described in US 6071285.

The balloons according to the invention may be formed using any suitable polymer materials known in the art. Both thermoplastic and thermosetting materials may be employed. Both elastomeric and non-elastomeric polymer materials may be employed.

Examples of suitable polymer materials include, but are not limited to, polyolefins, polyesters, polyethers, polyimides, polyamides, ionomeric polymers, polyurethanes, polycarbonates, polyvinyl chlorides, polyphenylene sulfides, block copolymer elastomers, and so forth. Balloon formation using polyimides are disclosed in U.S. Patent No. 6,024,722.

Some classes of materials, such as the polyurethanes, are available in both thermoplastic and thermosetting forms, as well as elastomeric and non-elastomeric versions.

More specific examples of useful polyolefins include, but are not limited to, polyethylenes and polypropylenes and any copolymers thereof.

Other specific examples of materials useful in making the balloons of the present invention include, but are not limited to, polyethylene terephthalate, polybutylene terephthalate, acrylonitrile-butadiene-styrene copolymers, polyether- polyester copolymers, copolyesters, polyether polyamide copolymers, polyether block amides, and so on and so forth.

The balloons may be formed using any suitable balloon forming methods known in the art. An example of one method is described in US 4,490,421.

The methods typically include the basic steps of extruding a tubular parison, placing the tubular parison in a balloon mold, and expanding the tubular parison into the desired balloon configuration in the balloon mold.

One typical method of balloon manufacture, involves placing an extruded polymeric tube in the mold cavity, and radially expanding the tube such that it fills the mold cavity.

In one embodiment, the balloon, when viewed in radial cross-section, is formed from at least one layer of a homogeneous polymer material or a homogeneous blend of polymer materials, which is extruded into a tube, the tube place into a mold, and then formed into a balloon by radially expanding the tube into the mold. As used herein, the term homogeneous shall be used to refer to a layer formed of a substantially uniform polymer composition.

A homogeneous layer may include fibers or particles mixed therein, providing that the fibers or particles are distributed substantially uniformly throughout the layer. For example, particles which are relatively small in size, for instance, those having a diameter of 1 micron or less, may be distributed therein.

A tube having multiple layers may also be employed. One method of forming a multilayer structure of this type is by coextrusion.

Furthermore, the tubular member may comprise fibers in the wall thereof, or fibers may be placed between a multilayer structure. Fibers may be in the form of a braid, weave, rove, web, etc., or may be randomly distributed in a homogeneous polymer layer as described above.

Any combination of such layers may be incorporated into the balloon structure, providing at least one layer is substantially homogeneous.

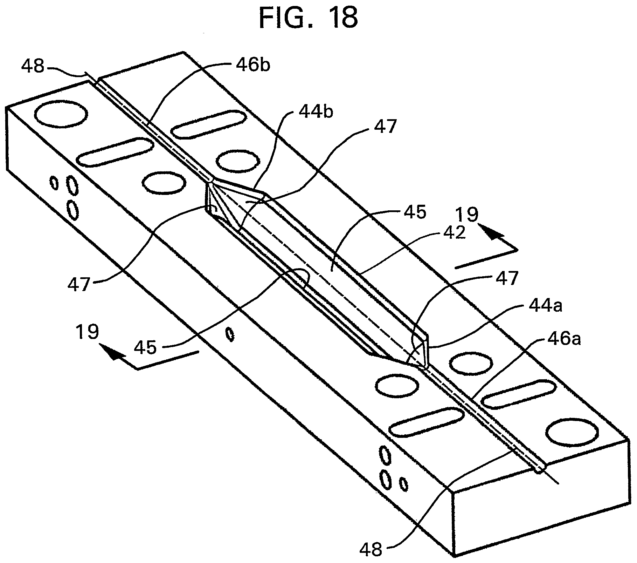

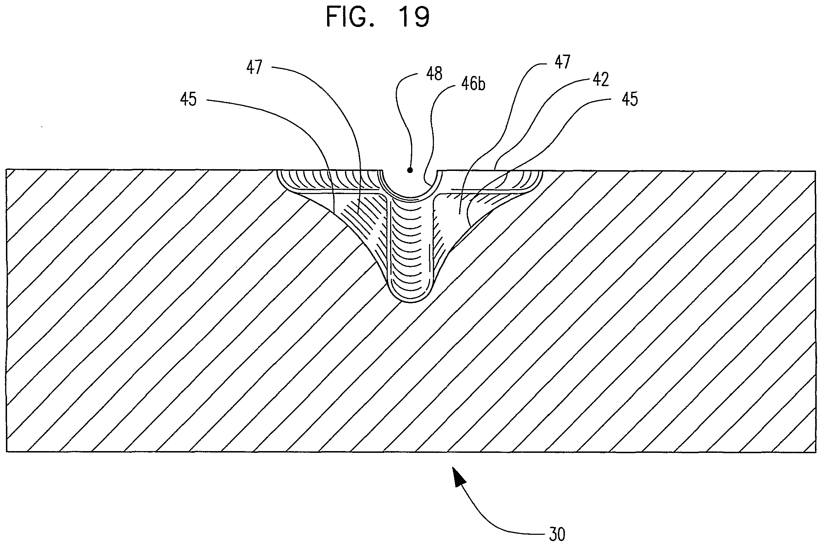

Desirably, the balloon is formed in a single molding step. FIG. 18 is a perspective view of one-half of a mold form 30 for manufacturing a balloon having concave side regions according to the invention. FIG. 19 is a radial cross-section taken at section 19 in FIG. 18. As can be seen from FIGS. 18 and 19, the mold form 30 is provided with the geometric configuration which is desired for the balloon in the static state, i.e. the state in which the balloon is released from the mold.

In the embodiment illustrated in FIG. 18, the mold cavity is shown having a central or body section 42, and first and second tapered sections 44a, 44b at either end of the central section 42, and end or waist sections 46a, 46b at either end of the tapered sections 44a, 44b.

Mold 30 has a plurality of concave side regions 45 extending at least along the central section 42 in a direction parallel to the longitudinal axis of the mold cavity, the concave regions 45 curving inward toward the longitudinal axis 48 of the mold 30, such that, in this particular embodiment, the central part of the concave side region is closest to the longitudinal axis. In this embodiment, each half of mold is formed with a cavity having two concave side regions 45. Please note, only one-half of the entire mold has been shown. The other half of the mold, would be a mirror image of the one shown in FIG. 18.

Concave side regions can be located in either or both of the tapered sections 44a, 44b as well as in the central section 42 of the mold cavity. In the embodiment shown in FIGS. 18 and 19, alternatively, each tapered cone section 44a, 44b, has a corresponding number of concave side regions 47 to the concave side regions 45 in the central section.

The outer wall of the resultant molded balloon is formed by (and corresponds to) the inner surface of the mold cavity. The resultant balloon will have a corresponding number of concave side regions to the entire mold cavity.

The balloon described above, is adapted for inflation from a folded configuration to an inflated, expanded configuration and back to a folded configuration. The concave side regions formed in the central section, and the tapered cone sections, facilitate folding and refolding of the expandable balloon member.

The balloons may further include partial or full coatings on the inner and/or outer walls. Examples of coating materials include, but are not limited to, those that provide lubricity, and those which may be included for purposes of drug delivery.

Any suitable lubricious material may be employed including both hydrophobic and hydrophilic lubricious materials. Examples of a hydrophobic lubricious material include, but are not limited to, silicone and fluoropolymers such as polytetrafluoroethylene.

Suitable examples of hydrophilic materials include, but are not limited to, polyalkylene oxides such as polyethylene oxide, polyvinyl pyrrolidone, polycarboxylic acids such as those based on maleic, fumaric or (meth)acrylic acid, for example.

Therapeutic agent(s) may be incorporated into a coating material, or other wise provided in combination with the balloons and catheter assemblies described herein. "Therapeutic agents," "drugs," "pharmaceutically active agents", "pharmaceutically active materials," and other related terms are employed in the art interchangeably. Hereinafter, the term therapeutic agent will be employed herein. Therapeutic agents include genetic materials, non-genetic materials, and cells. Of course mixtures of therapeutic agents may also be employed. Therapeutic agents are discussed in commonly assigned U.S. Patent Application 2004/0215169 and U.S. Patent No. 6855770.

Therapeutic agents may be incorporated into polymer coatings. Examples of suitable polymer coatings include both elastomeric and non-elastomeric polymer materials.

One suitable class of polymer coating materials includes block copolymer elastomers such as those having styrene end blocks. Examples include, but are not limited to, styrene-ethylene/propylene-styrene (SEPS), styrene-butadiene-styrene (SBS), styreneisoprene-styrene (SIS), styrene-ethylene/butylene-styrene (SEBS), styrene- isobutylene-styrene (SIBS), and so forth. Such polymer materials are disclosed in commonly assigned U.S. Patent Nos. 6855770 and 6545097

Other suitable polymer coating materials include, polyolefms, such as ethylene and propylene homopolymers, as well as any copolymers or terpolymers of ethylene and propylene such as ethylene-vinyl acetate copolymers, ethylene (meth)acrylate copolymers, ethylene n-butyl acrylate copolymers, and grafted polyolefins such as maleic anhydride grafted polyethylene or polypropylene.

Bioabsorbable polymers may also be employed as coating materials. Examples include, but are not limited to, poly(hydroxyvalerate), poly(L-lactic acid), polycaprolactone, poly(lactide-co-glycolide), poly(hydroxybutyrate), poly(hydroxybutyrate-co-valerate), polydioxanone, polyorthoesters, polyanhydrides, poly(glycolic acid), poly(D,L-lactic acid), poly(glycolic acid-co-trimethylene carbonate), polyphosphoesters, polyphosphoester urethanes, poly(amino acids), cyanoacrylates, poly(trimethylene carbonate), poly(iminocarbonate), copoly(ether- esters) (e.g. PEO/PLA), polyalkylene oxalates, polyphosphazenes and biomolecules such as fibrin, fibrinogen, cellulose, starch, collagen, hyaluronic acid, etc., and mixtures thereof. Bioabsorbable polymers are disclosed in U.S. Patent Nos. 6,790,228..

The therapeutic agents may be disposed within or under, the bioabsorbable polymer, for example.

The above lists are intended for illustrative purposes only, and are not intended to limit the scope of the present invention. Other materials not specifically listed herein, may be employed as well.

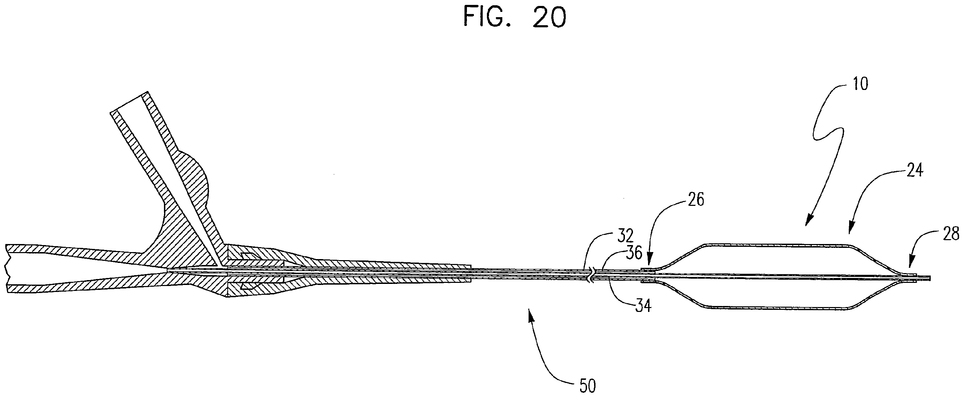

The balloons according to the present invention may be employed in combination with any suitable catheter assembly. Balloons are typically mounted on the distal end of the catheter assembly. For some catheter configurations known in the art, a dual shaft configuration including an inner shaft and an outer shaft is employed. An example of this type of catheter is shown as a longitudinal cross-sectional view in FIG. 20. The balloon is mounted at the distal end 24 of the catheter assembly 50 wherein the proximal end 26 of balloon 10 is mounted to the outer catheter shaft 32 and the distal end 28 of balloon 10 is mounted on the inner shaft 34.

Such catheter assemblies may further include additional features as are known in the art including, but not limited to, implantable stents which are employed in various vessels in the body, drug delivery, perfusion and dilatation features, or any combination of such features may be used in combination with the dilatation balloon of the present invention.

## Claims

1. A medical balloon (10) having a body section (16), a first tapered section and a second tapered section (14), waist sections (12), and a longitudinal axis, said balloon (10) having a static state, a deflated state and an inflated state, the balloon (10) configured and formed such that in the static state, at least the body section (16) of said balloon having a plurality of concave side regions (15) extending between the first tapered section (14) and the second tapered section (14), each concave side region (15) curving toward the longitudinal axis of the balloon (10), the balloon (10) comprising a plurality of atherotomes (22), each atherotome (22) located centrally in each concave side region (15), the body section having a circumference which when viewed in radial cross-section, has at least one layer extending around the entire circumference and each said layer is formed from a substantially homogeneous polymer material, and in the expanded state the body section (16) of said balloon is circular in cross-section; wherein the static state is the state of said balloon (10) as formed.

2. The balloon of claim 1 further having at least one expanded state, the body section (16) in the at least one expanded state having a substantially cylindrical configuration.

3. The medical balloon of claim 1 having three concave side regions.

4. The medical balloon of claim 1 having four concave side regions.

5. The medical balloon of claim 1, said at least one layer formed from at least one member selected from the group consisting of polyolefins, polyesters, polyethers, polyimides, polyamides, ionomeric polymers, polyurethanes, polycarbonates, polyvinyl chlorides, polyphenylene sulfides, styrenic block copolymers, and homogeneous mixtures thereof.

6. The medical balloon of claim 1, said at least one layer formed from polyethylene terephthalate, polybutylene terephthalate or a mixture thereof.

7. The medical balloon of claim 1 wherein the substantially homogeneous polymer material is a substantially homogeneous blend.

8. The medical balloon of claim 1 wherein the substantially homogeneous polymer layer further comprises fibers or particles which are distributed substantially uniformly throughout said at least one substantially homogeneous polymer layer.

9. The medical balloon of claim 1 comprising multiple layers.

10. The medical balloon of claim 9 wherein each of said multiple layers is substantially homogeneous.

11. The medical balloon of claim 1 further comprising a fiber layer.

12. The medical balloon of claim 1 further having a deflated state, and in the deflated state, the balloon having a number of wings, each of said wings corresponding to each of said plurality of concave side regions.

13. The medical balloon of claim 1 further comprising at least one coating, said coating comprises at least one member selected from the group consisting of polymers and therapeutic agents; and wherein said coating optionally comprises at least one lubricious polymer material.

## Patentansprüche

1. Medizinischer Ballon (10) mit einem Körperabschnitt (16), einem ersten konischen Abschnitt und einem zweiten konischen Abschnitt (14), Taillenabschnitten (12) und einer Längsachse, wobei der Ballon (10) einen statischen Zustand, einen entleerten Zustand und einen aufgeblasenen Zustand hat, der Ballon (10) im statischen Zustand so ausgebildet und geformt ist, dass zumindest der Körperabschnitt (16) des Ballons mehrere konkave Seitenbereiche (15) aufweist, die sich zwischen dem ersten konischen Abschnitt (14) und dem zweiten konischen Abschnitt (14) erstrecken, wobei sich jeder konkave Seitenbereich (15) zur Längsachse des Ballons (10) hin krümmt, der Ballon (10) mehrere Atherotome (22) aufweist, wobei sich die einzelnen Atherotome (22) jeweils in der Mitte der einzelnen konkaven Seitenbereiche (15) befinden, wobei der Körperabschnitt einen Umfang hat, der bei Betrachtung im radialen Querschnitt mindestens eine Schicht aufweist, die sich um den gesamten Umfang erstreckt, und jede solche Schicht aus einem im Wesentlichen homogenen Polymermaterial hergestellt ist, und wobei der Körperabschnitt (16) des Ballons im ausgedehnten Zustand einen kreisförmigen Querschnitt hat; wobei der statische Zustand der Zustand des Ballons (10) ist, in dem er gebildet wurde.

2. Ballon nach Anspruch 1, der ferner mindestens einen ausgedehnten Zustand aufweist, wobei der Körperabschnitt (16) in dem mindestens einen ausgedehnten Zustand im Wesentlichen zylindrisch ist.

3. Medizinischer Ballon nach Anspruch 1, der drei konkave Seitenbereiche aufweist.

4. Medizinischer Ballon nach Anspruch 1, der vier konkave Seitenbereiche aufweist.

5. Medizinischer Ballon nach Anspruch 1, wobei die mindestens eine Schicht aus mindestens einem Stoff ausgewählt aus der Gruppe bestehend aus Polyolefinen, Polyestern, Polyethern, Polyimiden, Polyamiden, ionomeren Polymeren, Polyurethanen, Polycarbonaten, Polyvinylchloriden, Polyphenylensulfiden, Styrolblockcopolymeren und homogenen Gemischen davon gebildet ist.

6. Medizinischer Ballon nach Anspruch 1, wobei die mindestens eine Schicht aus Polyethylenterephthalat, Polybutylenterephthalat oder einem Gemisch davon gebildet ist.

7. Medizinischer Ballon nach Anspruch 1, wobei das im Wesentlichen homogene Polymermaterial eine im Wesentlichen homogene Mischung ist.

8. Medizinischer Ballon nach Anspruch 1, wobei die im Wesentlichen homogene Polymerschicht ferner Fasern oder Partikel aufweist, die im Wesentlichen gleichmäßig in der mindestens einen im Wesentlichen homogenen Polymerschicht verteilt sind.

9. Medizinischer Ballon nach Anspruch 1, der mehrere Schichten aufweist.

10. Medizinischer Ballon nach Anspruch 9, wobei jede der mehreren Schichten im Wesentlichen homogen ist.

11. Medizinischer Ballon nach Anspruch 1, der ferner eine Faserschicht aufweist.

12. Medizinischer Ballon nach Anspruch 1, der ferner einen entleerten Zustand aufweist und in dem entleerten Zustand eine Anzahl von Flügeln hat, wobei jeder der Flügel jedem der mehreren konkaven Seitenbereiche entspricht.

13. Medizinischer Ballon nach Anspruch 1, der ferner mindestens eine Beschichtung aufweist, wobei die Beschichtung mindestens einen Stoff ausgewählt aus der Gruppe bestehend aus Polymeren und therapeutischen Mitteln aufweist und wobei die Beschichtung wahlweise mindestens ein gleitfähiges Polymermaterial aufweist.

## Revendications

1. Ballonnet médical (10) ayant une section de corps (16), une première section effilée et une seconde section effilée (14), des sections d'étranglement (12) et un axe longitudinal, ledit ballonnet (10) ayant un état statique, un état dégonflé et un état gonflé, le ballonnet (10) configuré et formé de telle sorte que, dans l'état statique, au moins la section de corps (16) dudit ballonnet ayant une pluralité de régions latérales concaves (15) s'étendant entre la première section effilée (14) et la seconde section effilée (14), chaque région latérale concave (15) s'incurvant en direction de l'axe longitudinal du ballonnet (10), le ballonnet (10) comprenant une pluralité d'athérotomes (22), chaque athérotome (22) situé en position centrale dans chaque région latérale concave (15), la section de corps ayant une circonférence qui, quand elle est observée en coupe transversale radiale, a au moins une couche s'étendant autour de toute la circonférence et chacune desdites couches est formée à partir d'un matériau polymère sensiblement homogène, et dans l'état expansé la section de corps (16) dudit ballonnet est circulaire en coupe transversale ; où l'état statique est l'état dudit ballonnet (10) tel qu'il est formé.

2. Ballonnet selon la revendication 1 ayant en outre au moins un état expansé, la section de corps (16) dans le au moins un état expansé ayant une configuration sensiblement cylindrique.

3. Ballonnet médical selon la revendication 1 ayant trois régions latérales concaves.

4. Ballonnet médical selon la revendication 1 ayant quatre régions latérales concaves.

5. Ballonnet médical selon la revendication 1, ladite au moins une couche formée à partir d'au moins un membre choisi dans le groupe consistant en les polyoléfines, les polyesters, les polyéthers, les polyimides, les polyamides, les polymères ionomères, les polyuréthanes, les polycarbonates, les poly(chlorures de vinyle), les poly(sulfures de phénylène), les copolymères séquencés styréniques et leurs mélanges homogènes.

6. Ballonnet médical selon la revendication 1, ladite au moins une couche formée à partir de polyéthylène téréphtalate, de polybutylène téréphtalate ou d'un mélange de ceux-ci.

7. Ballonnet médical selon la revendication 1 où le matériau polymère sensiblement homogène est un mélange sensiblement homogène.

8. Ballonnet médical selon la revendication 1 où la couche de polymère sensiblement homogène comprend en outre des fibres ou des particules qui sont distribuées de manière sensiblement uniforme dans toute ladite au moins une couche de polymère sensiblement homogène.

9. Ballonnet médical selon la revendication 1 comprenant de multiples couches.

10. Ballonnet médical selon la revendication 9 où chacune desdites multiples couches est sensiblement homogène.

11. Ballonnet médical selon la revendication 1 comprenant en outre une couche de fibres.

12. Ballonnet médical selon la revendication 1 ayant en outre un état dégonflé, et dans l'état dégonflé, le ballonnet ayant un certain nombre d'ailes, chacune desdites ailes correspondant à chacune de ladite pluralité de régions latérales concaves.

13. Ballonnet médical selon la revendication 1 comprenant en outre au moins un revêtement, ledit revêtement comprend au moins un membre choisi dans le groupe consistant en les polymères et les agents thérapeutiques ; et où ledit revêtement comprend éventuellement au moins un matériau polymère lubrifiant.
